# EUROPEAN PATENT APPLICATION

(11) **EP 0 811 684 A2**
(43) Date of publication of application: **10.12.1997**
(21) Application number: 97102130.8
(22) Date of filing: 09.10.1992
(51) Int. Cl.: C12N 15/12, C07K 14/72, G01N 33/68

(54) **Oxytocin receptor portion or variant, and dna coding for the same**

(30) Priority: 09.10.1991 JP 262167/91; 27.03.1992 JP 71258/92
(62) Divisional of application: 92309239.9
(71) Applicant: Rohto Pharmaceutical Co., Ltd., Osaka-shi, Osaka (JP)
(72) Inventor: Tanizawa, Osamu, Osaka-shi, Osaka (JP); Okayama, Hiroto, Bunkyo-ku, Tokyo (JP); Saji, Fumitaka, Nishinomiya-shi, Hyogo (JP); Azuma, Chihiro, Osaka-shi, Osaka (JP); Kimura, Tadashi, Osaka-shi, Osaka (JP)
(74) Representative: Schlich, George William

(57) **Abstract**

A DNA of at least 18 nucleotides encoding a portion or variant of an oxytocin receptor of at least 6 amino acids; cells transformed with a vector comprising the DNA; manufacture of portions or variants of an oxytocin receptor and use of the DNA to screen a cDNA library.

## Description

The present invention relates to a receptor of oxytocin being a posterior pituitary hormone which is widely used as an uterotonic agent in the general clinic field, a DNA sequence coding for the same, a recombinant DNA containing the DNA and a transformant having the recombinant DNA.

Oxytocin being a posterior pituitary hormone has an activity causing uterine contraction and is widely used for induction and enhancement of labor pains in the obstetric clinical field. However, there is a great individual variation in such activity. Also, oxytocin is effective against only the pregnant uterus in the term to show such activity. It has been considered that increase of the oxytocin receptor is essential for myometrium to obtain sensitivity to oxytocin.

Therefore, it is necessary to obtain an oxytocin receptor and a DNA sequence coding for the same, in order to minutely elucidate a series of biological response mechanism between oxytocin and the oxytocin receptor and an expression mode of oxytocin receptor on living tissue, further in order to enable to measure an expression level of the oxytocin receptor before labor or at the time of labor, construct screening method for evaluating oxytocin activity and relieve of labor pains by an antibody against the oxytocin receptor, and in order to obtain the oxytocin receptor, a DNA sequence coding for the same, a recombinant DNA containing the DNA and a transformant having the recombinant DNA. However, studies of isolation, purification and structural analysis of the oxytocin receptor are hardly carried out.

In consideration of such circumstance, the object of the present invention is to obtain an oxytocin receptor, a DNA sequence coding for the same, recombinant DNA containing the DNA and a transformant having the recombinant DNA.

According to the present invention there is provided an isolated oxytocin receptor comprising the amino acid sequence represented in SEQ ID NO:1, or portion or variant thereof.

In an embodiment of the invention the variant amino acid sequence differs from said amino acid sequence of SEQ ID NO: 1 in that one or more amino acids are substituted, added or deleted. In a further embodiment the variant amino acid sequence contains up to 4 amino acid substitutions, additions or deletions, and in a particular embodiment zero or one substitution, addition or deletion.

In another embodiment of the invention the substitutions to the amino acid sequence of SEQ ID NO: 1 are made according to the following table:-

| **ORIGINAL** **AMINO ACID** | **OPTIONAL** **SUBSTITUTE** |
|---|---|
| Ser | Cys |
| Arg | His, Lys |
| Leu | Ile, Met, Phe |
| Pro | Ala |
| Ala | Pro |
| Val | Met, Ile |
| Ile | Met, Leu, Phe, Val |
| Phe | Met, Tyr, Ile, Leu |
| Tyr | Phe |
| His | Gln, Arg |
| Gln | Glu, His |
| Asn | Asp |
| Lys | Arg |
| Asp | Asn |
| Glu | Gln |
| Met | Ile, Leu, Phe, Val |

In a still further embodiment of the invention the portion comprises a portion of the amino acid sequence of SEQ ID NO: 1 or of the variant of at least 350 amino acid residues in length, and in a particular embodiment a contiguous sequence of at least 350 amino acid residues contained within the sequence of SEQ ID NO: 1.

According to a second aspect of the invention there is provided a DNA segment coding for an oxytocin receptor, or portion or variant thereof. In an embodiment of the second aspect of the invention, the DNA segment encodes the amino acid sequence given in SEQ ID NO: 1 or portion thereof of at least six amino acids in length. In a further embodiment of the second aspect of the invention, the segment has the nucleotide sequence represented in SEQ ID NO: 2 or portion thereof of at least 18 nucleotides in length.

In a still further embodiment of the second aspect of the invention the variant differs from the DNA sequence shown in SEQ ID NO: 2 in that one or plural nucleotides are substituted, added or deleted.

In a still further embodiment of the second aspect there is provided a DNA segment which is at least 75% homologous with the sequence of SEQ ID NO: 2, and in a particular embodiment is substantially homologous with the sequence of SEQ ID NO: 2.

According to a third aspect of the invention there is provided a recombinant DNA molecule comprising a vector and a DNA segment according to any of the embodiments of the second aspect of the invention.

According to a fourth aspect of the invention there is provided a DNA segment that hybridizes to a DNA segment according to any of the embodiments of the second aspect of the invention.

According to a fifth aspect of the invention there is provided a cell transformed with a DNA molecule according to the third aspect of the invention.

According to a sixth aspect of the invention there is provided a method of producing an oxytocin receptor comprising culturing cells according to the fifth aspect of the invention under conditions such that said DNA segment is expressed so that said oxytocin receptor is produced, and isolating said oxytocin receptor.

According to a seventh aspect of the invention there is provided a kit comprising at least one of an oxytocin receptor or antibody specific therefore disposed within a container means, and a buffer.

As the result of the continuous effort to obtain an oxytocin receptor of the present inventors, it has been found that as a membrane current can be high-sensitively detected the phenomenon wherein a protein encoded by a specific mRNA increases intracellular calcium ion in a perfusion with oxytocin and causes influx of chlorine ion on the oocytes of African clowed toad, Xenopus laevis. Usually a mRNA fractioned by means of sucrose density gradient method for the like is used. However, the starting material for the mRNA is a human pathologic specimen in an extremely specific acosmia, and an amount of the material is finite. It is impossible to take the material again. Therefore, the present inventors have succeeded in cloning a gene coding for a biologically active protein causing membrane current in perfusion with' oxytocin on cell membrane, i.e. oxytocin receptor, for the first time by means of preparing cDNA library from whole mRNA by using a plasmid vector and then fractioning the library by size. Consequently the present invention is accomplished.

Hereinafter, the oxytocin receptor, the DNA sequence, the recombinant DNA and the transformant of the present invention are explained.

The oxytocin receptor of the present invention is a polypeptide or protein comprising the whole or a part of the amino acid sequence shown in SEQ ID NO: 1, or a variant thereof, and a biologically active polypeptide or protein causing increase of concentrations of intracellular calcium ion and inositol triphosphate. More particularly, the oxytocin receptor of the present invention is a polypeptide or protein having an amino acid sequence wherein one or plural amino acid residues in the amino acid sequence shown in SEQ ID NO: 1 are substituted, or one or plural amino acid residues are lacking, further, or one or plural amino acid residues are added to the amino acid sequence shown in SEQ ID NO: 1.

The DNA sequence of the present invention is a DNA sequence coding for the oxytocin receptor of the present invention. Examples of the DNA sequence of the present invention are, for instance, a DNA sequence comprising the whole or a part of the base sequence shown in SEQ ID NO: 2, or a varied DNA thereof. More particularly, there can be exemplified a DNA sequence which has a DNA sequence wherein one or plural nucleotides in DNA sequence shown in SEQ ID NO: 2 are substituted with other nucleotides, or one or plural nucleotides are lacking, further, or one or plural nucleotides are added to the DNA sequence shown in SEQ ID NO: 2.

The recombinant DNA of the present invention is a DNA containing the DNA sequence of the present invention in a suitable expression vector. The recombinant DNA of the present invention may contain a sequence for regulating an expression which is functionally combined with the DNA sequence of the present invention. The vector used in the present invention is not particularly limited, when the vector can be replicated in a suitable host and can be expressed. As examples of the vector used in the present invention, pcD vector, the varied pcD vector explained in Example 1 described below, can be exemplified.

The present invention is more specifically described and explained by means of the following Example. It is to be understood that the present invention is not limited to the Example.

### Example 1

Human myometrium tissue which could be accidentally obtained (pathologic specimen of the case wherein extensive bleeding was encountered by uterine rupture just after the vaginal delivery and the whole uterus was obliged to removed) was rapidly freezed in liquid nitrogen and stored at -70°C.

### (1) Size estimation of mRNA coding for the oxytocin receptor

The human myometrium tissue was powdered in liquid nitrogen. The powdered tissue was homogenized in a solution containing 5.5 M guanidium isothiocyanate, 100 mM 2-mercaptoethanol, 0.5 % sodium N-laurylsarcosine and 2.5 mM sodium citrate (pH 7.0), layered onto the cushion of the solution containing 5.7 M CsCℓ and 0.1 M EDTA (pH 7.0) and ultracentrifuged to recover whole RNA as precipitation. Thus obtained precipitation of whole RNA was purified by repeating precipitation with ethanol. From the whole RNA was isolated polyadenyrated RNA by using oligo-dT cellulose column.

In the solution containing 10 mM Tris-HCℓ and 1 mM EDTA (pH 7.0) was dissolved 160 µg of the polyadenyrated RNA. After performing heat-denaturation, the solution with 0.1 % sodium N-laurylsarcosine was layered onto the discontinuous sucrose density-gradient (8 layers, 5 %-25 % sucrose) and ultracentrifuged at 35,000 rpm for 7.5 hours. The ultracentrifuged layers were fractionated from low density of sucrose by means of a fraction collecter to give 25 fractions of 0.4 mℓ. Each size fractionated mRNA was recovered by precipitation with ethanol. The whole mRNA was dissolved in water in a ratio of 1 mg/mℓ, and the size fractionated mRNA was dissolved in 5 µ ℓ of water, to give samples for microinjection into oocytes of Xenopus. The samples were injected into the Xenopus oocytes treated at room temperature for 3 hours with the modified Barth's medium from which calcium ion was removed (refer to Transcription and Translation Edt. B. Hames, S. Higgins IRL Press. 1984. Oxford, pp 271-301) wherein 2 mg/mℓ collagenase was dissolved. Into the Xenopus oocytes was injected 50 nℓ of each size fractionated mRNA by means of a hydraulic microsyringe equipped with a microcapillary. These oocytes were cultured in the modified Barth's medium at 19°C for 72 hours.

Then, according to the method of Dascal et al. (refer to J. Physiol 366, 299-313, (1985)), into these oocytes were inserted 2 microelectrodes filled with 3M KCℓ. One electrode was electrified to settle a membrane potential to -60 mV. The other was attached to a system for measuring a membrane current. This oocyte had been perfused with Normal Ringer's Solution (115 mM NaCℓ, 1 mM KCℓ, 1.8 M CaCℓ ₂ and 5 mM Tris pH 7.4) and then perfused with 1 µM oxytocin for only 1 minute. In case of 1 µ M oxytocin, a membrane current was observed in the both oocyte into which the whole mRNA was injected and the oocyte into which the 19th to the 21st fractions of the size fractionated mRNA was injected. It is inferred that an oxytocin receptor capable of binding to oxytocin was expressed on the oocytes because a membrane current was caused by the perfusion with oxytocin. That is, such fact indicates that a mRNA of about 4-5 kb (killbases) capable of showings - activity of the oxytocin receptor exists in human myometrium tissue in term.

### (2) Preparation of cDNA library

According to the method of Okayama et al. (refer to Methods in Enzymology 154, 3-28, (1987)), the whole RNA of human myometrium was purified. From the purified RNA was obtained the whole mRNA by means of an oligo(dT)-cellulose column.

As a vector for preparing a library was used a varied vector produced by M. Brownstein of NIH, U.S.A. (unpublished). The varied vector is a vector obtained by improving the expression vector pcD for animal cells developed by Okayama-Berg (refer to Hiroto Okayama and Paul Berg "Molecular cell Biology" 3, 280 (1983) and 2, 161 (1982)). The varied vector is the vector primer pcDV1 modified by inserting T7 RNA polymerase promoter and sites to be cleaved by restriction enzymes NotI, BstXI and XbaI therein. The linker used in the present invention is the linker pL1 wherein promoter region of SP6 RNA polymerase was inserted.

This vector primer and linker were provided by Mr. Brownstein of NIH, U.S.A. in a condition that to the vector primer and the linker were added dG tail and dT tail, respectively by means of terminal deoxynucleotidyl transferase (TdT) and then purified by means of a low melting agarose and oligo(dA)-cellulose column.

By using 7 µg of the purified whole mRNA was synthesized cDNA of the first strand according to the above-mentioned Methods in Enzymology, 154, 3-28, (1987). The cDNAs of the average length 630 bp were synthesized by this reaction. Then, this cDNA was tailed with dC by reacting with 10 units of TdT at 37°C for 5 minutes in the presence of dCTP. Thus obtained mRNA-cDNA hybrid vector primers were digested and reacted with 28 units HindIII at 37°C for 1 hour.

Successively, the 1/10 amount of this mRNA-cDNA hybrid vector primers digested with HindIII were mixed and annealed with 0.25 pM the varied pL1 linker which was previously prepared. After annealing, the hybrid vector primers with the linker were looped by reacting with 100 units of DNA ligase (derived from E. coli) in the presence of 0.1 mM β -NAD at 12°C overnight. The looped mRNA-cDNA hybrid was reacted in the presence of 40 µ M dNTP with 30 units of RNase H, 50 units of DNA ligase (derived from E. coli) and 3 units of DNA polymerase I at 12°C for 60 minutes, and successively at room temperature for 60 minutes to be converted into a double-stranded cDNA. Thereby cDNA library derived from human myometrium was constructed in the varied pcD vector.

Subsequently cDNA library was transformed into a high efficience competent cells of E. coli DH5 which was prepared according to the method developed by Inoue and Okayama (refer to Gene 96, 23-28 (1990)) to give 4 x 10⁷ cells of independent transformants. After proliferating this transformants in L-Broth medium containing 100 µ g/mℓ ampicillin, plasmids were isolated to obtain a plasmid library according to a standard method for preparing plasmid DNA.

### (3) Preparation of sublibrary

As described in (1), cDNA coding for an oxytocin receptor is about 4 to 5 kbp or so. The varied pcD vector is about 3 kbp. Therefore, with 80 units of each of NotI and PvuI was digested 40 µg of each this cDNA library, respectively, and thus obtained linear cDNA was separated into each linear cDNA having a length of from 7 kbp to 23 kbp by electrophoresis using low melting agarose gel. Each of thus obtained separated linear cDNA was reacted with 1.2 units of T4 DNA ligase in the presence of 66 µ M ATP in a reaction system of 200 µ ℓ at 4°C overnight to be looped again. The sublibrary (NotI sublibrary), which was looped again after digesting with NotI, contained 60,000 to 90,000 clones. The sublibrary (PvuI sublibrary), which was looped again after digesting with PvuI, contained about 600,000 clones.

### (4) Transcription in vitro

### (a) Preparation of template DNA

The 1/20 amount of the NotI sublibrary and the 1/200 amount of the PvuI sublibrary, which were obtained in (3), were introduced into DH5 respectively to give transformants. As to these transformants, 8 pools respectively, 16 pools in total were proliferated in 50 mℓ of L-Broth medium containing 100 µ g/mℓ ampicillin until the OD⁶⁰⁰ becomes about 1 (2 mℓ of each culture solution was taken and freezed for preservation in the presence of 7 % DMSO). Thereafter 7.5 mg of chloramphenicol was added to the culture solution and further culturing was performed for 16 hours. The cells were collected, and plasmids were isolated according to a standard method for preparing plasmid DNA. Ten µg of each pool prepared from the NotI sublibrary (N1-N8) were digested with 12 units of NotI at 37°C for 60 minutes to give linear DNAs. Also, 10 µg of each pool prepared from the PvuI sublibrary (P1-P8) were digested with 12 units of BstXI at 50^{°}C for 90 minutes to give linear DNAs. These were purified by a conventional treatment with phenol/chloroform and precipitation with ethanol. The purified cDNAs were dissolved in a solution containing 10 mM Tris (pH 7.0) and 1 mM EDTA so as to give a concentration of 1 µ g/µ ℓ. Thus obtained solution was used as a solution of template DNA.

### (b) Transcription in vitro

Five units of SP6 RNA polymerase (Bethesda Research Laboratories) was added to 25 µ ℓ of the reaction liquid consisting of the constitution described below (40 mM Tris (pH7.9), 6 mM MgCℓ₂ and 2 mM spermidine hydrochloride as a buffer, and a substrate (a mixture of ATP, CTP, GTP and UTP), m7G (5') ppp (5')G and DTT of a mRNA caping kit of Stratagen Cloning Systems were used). Then, using 5 µ g of each template DNA obtained in (a), the mixture was reacted at 39°C for 90 minutes. Further, thereto was added 10 units of RNase-free DNaseI (Stratagen Cloning Systems) and the reaction was carried out at 37°C for 5 minutes to decompose the template DNA. Successively, the reaction liquid was applied to Sephadex G-50 spun column (Boehringer Mannheim GmbH). The reaction liquid was passed through the column by centrifuging at 2200rpm for 5 minutes and an excess substrate was removed. Further this reaction liquid was purified by a conventional treatment with phenol/chloroform and precipitation with ethanol. About 2-3 µ g of each synthesized mRNA was recovered.

### (5) Measurement of oxytocin receptor activity

In 2.5 µ ℓ of water was dissolved each of 16 pools of the synthesized mRNA obtained in (4)-(b). By means of the technique described in (1), thus obtained solution was injected into 15-20 oocytes of Xenopus per pool. The oocytes. were cultured at 19°C for 3 days in the modified Barth's medium. Then, according to the method described in (1) these oocytes into which the mRNA derived from each pool were injected were perfused, and change of a membrane current was examined in case of perfusing with 1 µ M oxytocin for 1 minute. In the oocytes into which one pool derived from the NotI sublibrary among the pools prepared in (4) was injected, was detected a membrane current caused by oxytocin. On the ground that a membrane current was caused by perfusion with oxytocin, it is inferred that in an oxytocin receptor capable of binding to oxytocin is expressed in the oocyte. Because the pool consisted of about 6,000 clones, a part of the cells being obtained and stored in (4)-(a) were diluted, and 8 pools containing 1,500 clones were prepared. According to the method described in (4), a template DNA was prepared, and a synthesized mRNA was prepared by transcription in vitro. Subsequently, screening wherein injection into Xenopus oocytes was performed, was repeated again. As a result, 4 positive pools as to an oxytocin receptor activity were obtained. These positive pools were selected by the above-mentioned screening to finally give 2 clones showing an oxytocin receptor activity. As the result of digestion with restriction enzymes, these two clones were found to be identical. The length of cDNA thereof was about 4 kbp.

This cDNA was subjected to sequencing according to dideoxy method of Sanger et al. Amino acid sequence deduced from the determined base sequence (this cDNA is in a form of double strand) is shown in SEQ ID NO: 1.

### (6) Study of specificity of expressed oxytocin receptor

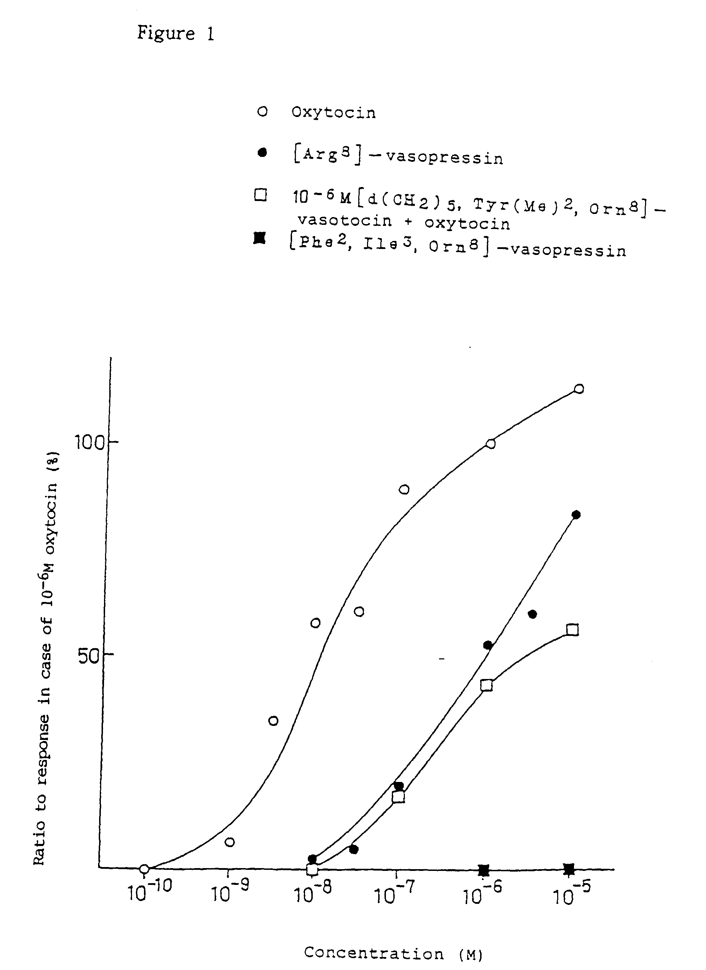

The single clone of cDNA of an oxytocin receptor obtained in (5) was again amplified and purified in a form of plasmid. Then, 10 µg of the plasmid was digested with ristriction enzyme NotI to give a linear DNA. A synthesized mRNA was obtained using 1 µg of the linear DNA as a template according to the method described in (4). The mRNA was injected into Xenopus oocyte in amount of 2-5 ng per oocyte, and the injected oocytes were cultured. These oocytes were perfused with each of 10⁻¹⁰M-10⁻⁵M oxytocin and 10⁻⁸M-10⁻⁵M Arg⁸⁻vasopressin for 1 minute, and dimensions of the membrane current thereof were compared. Each ratio of a current to the detected current during the perfusion with 10⁻⁶M oxytocin regarded as 100 % was shown in Fig. 1. As is clear from Fig. 1, Arg⁸⁻vasopressin could not cause approximately the same membrane current in case of oxytocin until the concentration in perfusion is nearly hundred times as high as that of oxytocin. Also, as to concentrations of both substances required so as to cause a current equal to 50 % of the largest current, in case of oxytocin, the concentration was about 10⁻⁸M and close to a physiological concentration. In contrust, that of the vasopressin was about 10⁻⁶M. Therefore, it was found that the cDNA isolated by us is a cDNA coding for a receptor specific to oxytocin. Also, in case of previously perfusing for 1 minute with 10⁻⁶M vasotocin known to be a specific antagonist to oxytocin ([d(CH₂)₅, Tyr(Me)², Orn⁸]-vasotocin) (refer to K. Bankowski et al., Int. J. Peptide Protein Res. 16, 382 (1980)) just before perfusion with oxytocin, a membrane current caused immediately thereafter by perfusion with oxytocin decreased to not more than 50 % of the largest current in case of 10⁻⁶M oxytocin. Further, using 10⁻⁶M or 10⁻⁵M of [Phe², Ile³, Orn⁸]-vasopressin being a vasopressin selective agonist, no response was observed. Such observations indicate that cDNA obtained by us codes for a receptor capable of specifically reacting with oxytocin and binding to oxytocin.

Because there were obtained the receptor, the DNA sequence, the recombinant DNA and the transformant of the present invention:
1) It comes to be possible to infer labor time from expression level of an oxytocin receptor by using uterine endometrium cells by reason that oxytocin receptors increase on uterine endometrium cells just before delivery.
2) It comes to be possible to scientifically and semi-quantitativelly predict whether or not relief of labor pains of patients with preterm labor is possible by reason that oxytocin receptors increase even in the preterm labor cases.
3) Prevention of hypertonic uterine dysfunction or determination of time of inducing labor pains can be carried out on the objective grounds by previously estimating oxytocin receptor level before using an agent inducing labor pains.
4) Expression of the receptor of the present invention in an animal cells or a Xenopus oocyte enables screening for estimation of an oxytocin activity.
5) Lelief of labor pains comes to be possible by preparing an antibody against the receptor of the present invention and inhibiting binding to oxytocin thereby.

### BRIEF DESCRIPTION OF THE DRAWINGS

### Figure 1

Figure A graph showing concentration of substances perfused on a cross axis (concentration of oxytocin in case of 10⁻⁶M vasotocin + oxytocin) and each ratio of a current to the detected current during the perfusion with 10⁻⁶M oxytocin regarded as 100 % on a vertical axis.

The clones referred to at (5) above, that is, JM 103 derived from E.coli K12 into which recombinant DNA was introduced, have been deposited with the Fermentation Research Institute (Deposit No. Bikouken 12907 (FERM P-12907)).

SEQUENCE ID NO: 1

SEQUENCE LENGTH : 389 amino acids
SEQUENCE TYPE : Amino acid
MOLECULE TYPE : Protein
HYPOTHETICAL SEQUENCE : Yes
ORIGINAL SOURCE ORGANISM : Homo Sapiens
TISSUE TYPE : Myometrium tissue
SEQUENCE


1               5                   10                  15

            20                  25                  30

        35                  40                  45

    50                  55                  60

65                  70                  75                  80

                85                  90                  95

            100                 105                 110

        115                 120                 125


    130                 135                 140

145                 150                 155                 160

                165                 170                 175

            180                 185                 190

        195                 200                 205

    210                 215                 220

225                 230                 235                 240

                245                 250                 255

            260                 265                 270

275 280 285

290 295 300

305 310 315 320

325 330 335

340 345 350


355 360 365

370 375 380

385
SEQ ID NO: 2

SEQUENCE LENGTH : 1167 nucleotides
SEQUENCE TYPE : nucleic acid
STRANDENESS : double stranded
MOLECULE TYPE : cDNA to mRNA
HYPOTHETICAL SEQUENCE : No
ORIGINAL SOURCE ORGANISM : Homo Sapiens
TISSUE TYPE : Myometrium tissue
SEQUENCE
ATG GAG GGC GCG CTC GCA GCC AAC TGG AGC GCC GAG GCA GCC AAC GCC 48

1               5                   10                  15
AGC GCC GCG CCG CCG GGG GCC GAG GGC AAC CGC ACC GCC GGA CCC CCG 96

            20                  25                  30
CGG CGC AAC GAG GCC CTG GCG CGC GTG GAG GTG GCG GTG CTG TGT CTC 144

        35                  40                  45
ATC CTG CTC CTG GCG CTG AGC GGG AAC GCG TGT GTG CTG CTG GCG CTG 192

    50                  55                  60
CGC ACC ACA CGC CAG AAG CAC TCG CGC CTC TTC TTC TTC ATG AAG CAC 240

65                  70                  75                  80

CTA AGC ATC GCC GAC CTG GTG GTG GCA GTG TTT CAG GTG CTG CCG CAG 288

                85                  90                  95
TTG CTG TGG GAC ATC ACC TTC CGC TTC TAC GGG CCC GAC CTG CTG TGC 336

            100                 105                 110
CGC CTG GTC AAG TAC TTG CAG GTG GTG GGC ATG TTC GCC TCC ACC TAC 384

        115                 120                 125
CTG CTG CTG CTC ATG TCC CTG GAC CGC TGC CTG GCC ATC TGC CAG CCG 432

    130                 135                 140
CTG CGC TCG CTG CGC CGC CGC ACC GAC CGC CTG GCA GTG CTC GCC ACG 480

145                 150                 155                 160
TGG CTC GGC TGC CTG GTG GCC AGC GCG CCG CAG GTG CAC ATC TTC TCT 528

                165                 170                 175
CTG CGC GAG GTG GCT GAC GGC GTC TTC GAC TGC TGG GCC GTC TTC ATC 576

            180                 185                 190
CAG CCC TGG GGA CCC AAG GCC TAC ATC ACA TGG ATC ACG CTA GCT GTC 624

        195                 200                 205
TAC ATC GTG CCG GTC ATC GTG CTC GCT ACC TGC TAC GGC CTT ATC AGC 672

    210                 215                 220
TTC AAG ATC TGG CAG AAC TTG CGG CTC AAG ACC GCT GCA GCG GCG GCG 720

225                 230                 235                 240

GCC GAG GCG CCA GAG GGC GCG GCG GCT GGC GAT GGG GGG CGC GTG GCC 768

                245                 250                 255
CTG GCG CGT GTC AGC AGC GTC AAG CTC ATC TCC AAG GCC AAG ATC CGC 816

            260                 265                 270
ACG GTC AAG ATG ACT TTC ATC ATC GTG CTG GCC TTC ATC GTG TGC TGG 864

        275                 280                 285
ACG CCT TTC TTC TTC GTG CAG ATG TGG AGC GTC TGG GAT GCC AAC GCG 912

    290                 295                 300
CCC AAG GAA GCC TCG GCC TTC ATC ATC GTC ATG CTC CTG GCC AGC CTC 960

305                 310                 315                 320
AAC AGC TGC TGC AAC CCC TGG ATC TAC ATG CTG TTC ACG GGC CAC CTC 1008

                325                 330                 335
TTC CAC GAA CTC GTG CAG CGC TTC CTG TGC TGC TCC GCC AGC TAC CTG 1056

            340                 345                 350
AAG GGC AGA CGC CTG GGA GAG ACG AGT GCC AGC AAA AAG AGC AAC TCG 1104

        355                 360                 365

TCC TCC TTT GTC CTG AGC CAT CGC AGC TCC AGC CAG AGG AGC TGC TCC 1152

    370                 375                 380
CAG CCA TCC ACG GCG 1167

385

## Claims

1. An isolated DNA segment encoding a portion or a variant of an oxytocin receptor, said portion or variant comprising at least 6 and up to but not including 350 contiguous amino acids from SEQ ID NO: 1.

2. An isolated DNA segment comprising at least 18 and up to but not including 1050 contiguous nucleotides from SEQ ID NO: 2.

3. A recombinant DNA molecule comprising a vector and a DNA segment according to Claim 1 or 2.

4. A cell transformed with a DNA molecule according to Claim 3.

5. A method of producing a portion or a variant of an oxytocin receptor comprising culturing cells according to Claim 4 under conditions such that said DNA segment is expressed and said portion or variant of said oxytocin receptor is produced, and isolating said portion or said variant.

6. A method of identifying a DNA encoding an oxytocin receptor or a portion or variant thereof, comprising screening a cDNA library with a DNA according to Claim 1 or 2.

7. Use of a DNA according to Claim 1 or 2 in manufacture of a probe for screening a cDNA library.
